# EUROPEAN PATENT APPLICATION

(11) **EP 2 642 021 A1**
(43) Date of publication of application: **25.09.2013**
(21) Application number: 11841647.8
(22) Date of filing: 17.11.2011
(51) Int. Cl.: D06M 15/03, D06M 15/15

(54) **PROTEOGLYCAN-BONDED FIBER PRODUCT AND METHOD OF MANUFACTURING SAME**

(30) Priority: 19.11.2010 JP 2010258545
(71) Applicant: Otsuka Pharmaceutical Co., Ltd., Tokyo 101-8535 (JP)
(72) Inventor: TAKI, Takao, Osaka-shi Osaka 541-0045 (JP)
(74) Representative: HOFFMANN EITLE
(86) International application number: PCT/JP2011/076571
(87) International publication number: WO 2012/067201

(57) **Abstract**

An objective is to provide fiber products having smooth texture and excellent softness, and in addition, excellent durability. A proteoglycan-bonded fiber product including a fiber to which a proteoglycan is covalently bonded is provided.

## Description

### Technical Field

The present invention relates to proteoglycancovalently-bonded fiber products including a fiber to which a proteoglycan is covalently bonded, and to methods for manufacturing the products.

### Background Art

Proteoglycans are glycoconjugates consisting of a core protein and glycosaminoglycans (mucopolysaccharides) attached thereto. Proteoglycans are the major components of cellular surfaces and extracellular matrices, and occur in skin tissue, cartilage tissue, bone tissue, vascular tissue, and the like. Proteoglycans are known to have moisture-retentive and antiinflammatory properties.

On the other hand, there have been a wide variety of consumer preferences for fiber products, such as handkerchiefs, towels, clothes, and the like. To satisfy such consumer preferences, fiber products which have various additional or modified functions have been proposed and developed. For example, it has been proposed that proteoglycans or other plant extracts are firmly attached to a fiber in attempts to increase the water absorbability of the fiber or prevent buildup of static charge on the fiber (PTL 1).

PTL 1 discloses manufacture of a fiber product modified as follows. A fiber is immersed in a treatment solution containing a low molecular weight hydrolyzed protein having an average molecular weight of 2,000 to 6,000, a high molecular weight hydrolyzed protein having an average molecular weight of 10,000 to 80,000, and a cross-linking agent and a functional material, and the excess treatment solution is squeezed out of the fiber, and moisture is then removed by drying, followed by a thermal treatment to cure a binder.

### Citation List

### Patent Literature

PTL 1: Japanese Patent No. 3038202

### Summary of Invention

### Technical Problem

However, in fiber products obtained by hitherto known techniques, such as that proposed in PTL 1, a binder is used to attach a functional material to a fiber, leading to hard, stiff, and rough texture, which does not make the product very satisfactorily comfortable in use. Also, in fiber products obtained by hitherto known techniques, the functional material comes off the fiber due to repeated washing, likely leading to a reduction in modified quality or durability.

In view of the above situation, an object of the present invention is to provide a functional fiber product which has smooth texture and excellent softness, and excellent durability.

### Solution to Problem

The present inventors have conducted extensive research in order to solve the above problems. As a result, the present inventors have found that a functional fiber product having smooth texture and excellent softness, and excellent durability, is obtained by bonding a proteoglycan directly to a fiber, i.e., covalently bonding a proteoglycan to a fiber, instead of attaching a functional material, such as a proteoglycan or the like, to a fiber using a cross-linking agent and a hydrolyzed protein as disclosed in PTL 1. The present inventors have conducted further studies and modifications based on such a finding to achieve the present invention.

Representative aspects of the present invention are illustrated as follows.
Aspect 1: A proteoglycan-bonded fiber product including a fiber to which a proteoglycan is covalently bonded.
Aspect 2: A proteoglycan-bonded fiber product including a fiber to which a proteoglycan is directly covalently bonded.
Aspect 3: The proteoglycan-bonded fiber product of aspect 1 or 2, in which the fiber is a cellulose-based fiber.
Aspect 4: The proteoglycan-bonded fiber product of aspect 2 or 3, in which the covalent bond is formed by a reaction of
   (a) an amino group of a core protein of the proteoglycan, and
   (b) an aldehyde group of at least one acyclic glucose constituting the cellulose-based fiber.
Aspect 5: The proteoglycan-bonded fiber product of any of aspects 1 to 4, in which the proteoglycan is aggrecan.
Aspect 6: A method for manufacturing a proteoglycan-bonded fiber product, including the step of covalently bonding a proteoglycan to a fiber.
Aspect 7: The method of aspect 6, in which the step of covalently bonding a proteoglycan to a fiber includes the following sub-steps of
   (1) treating a cellulose-based fiber with an oxidant to open the ring of a glucose molecule constituting the cellulose fiber to form an aldehyde group, and
   (2) reacting the cellulose-based fiber having the aldehyde group obtained by step (1) with the proteoglycan in the presence of a reducing agent.
   Aspect 8: The method of aspect 7, further including the step of
   bonding, one or more times, an additional proteoglycan to the proteoglycan of the proteoglycan-bonded fiber product obtained by sub-step (2).

### Advantageous Effects of Invention

The proteoglycan-bonded fiber product of the present invention has smooth texture and excellent softness, and in addition, excellent durability which does not allow such feel in use to be readily lessened, even if the product is repeatedly used or washed. The proteoglycan-bonded fiber product of the present invention has moisture retentive properties derived from the proteoglycan, and therefore, can provide moisture to the skin. The manufacturing method of the present invention can efficiently manufacture the fiber product having excellent feel in use and durability.

### Description of Embodiments

The present invention will be described in detail below.

The present invention provides a proteoglycan-bonded fiber product including a fiber to which a proteoglycan is covalently bonded.

Proteoglycan is the collective name of molecules in which one or more glycosaminoglycan chains are covalently bonded to a core protein. Examples of a glycosaminoglycan bonded to a proteoglycan used in the present invention include chondroitin sulfate, dermatan sulfate, heparan sulfate, and keratan sulfate.

Proteoglycans are classified, depending on the type of the glycosaminoglycan bonded to the core protein, as chondroitin sulfate proteoglycans, dermatan sulfate proteoglycans, heparan sulfate proteoglycans, keratan sulfate proteoglycans, and the like. The proteoglycan used in the present invention may be any of those proteoglycans. Alternatively, proteoglycans are classified, depending on the origin or function, as aggrecan, versican, neurocan, brevican, decorin, biglycan, serglycin, fibromodulin, perlecan, syndecan, glypican, lumican, and keratocan. Although any of those proteoglycans can be used in the present invention, chondroitin sulfate proteoglycans is preferable, and aggrecan is more preferable.

The origin of the proteoglycan used in the present invention is not particularly limited. The proteoglycan used in the present invention may be derived from any animals including: mammals, such as humans, cattle, pigs, and the like; birds, such as chickens and the like; fish, such as sharks, salmons, and the like; crustaceans, such as crabs, shrimps, and the like; cnidarians, such as jellyfishes and the like; and the like. Of these origins, proteoglycans derived from mammals such as pigs and the like and fish such as salmons and the like are preferable in terms of ease of availability, moisture retention, and smooth texture provided when bonded to a fiber, proteoglycans derived from fish are more preferable, proteoglycans derived from salmons are particularly preferable, and proteoglycans derived from the nasal cartilages of salmons are most preferable.

The molecular weight of the proteoglycan used in the present invention is not particularly limited, and is suitably set. A preferable molecular weight of the proteoglycan is several tens of thousands to three million, preferably several hundreds of thousands to one million, and more preferably four hundred thousand to five hundred thousand.

The fiber in the present invention is a component of the fiber product, and is not particularly limited, as long as it can form covalent bond with the proteoglycan. The fiber can be suitably selected from various fibers and used, depending on the type or use of the fiber product. The fiber used in the present invention may be either a naturally-occurring fiber or a chemical fiber (artificial fiber).

Examples of the naturally-occurring fiber which can be used in the present invention include plant fibers, animal fibers, and mineral fibers. Examples of the chemical fiber include inorganic fibers, regenerated fibers, semi-synthetic fibers, and synthetic fibers. Examples of the plant fiber include cotton, kapok, hemp, palm fiber, soft rush, straw, and the like. Examples of the animal fiber include silk, sheep wool, goat wool, cashmere, mohair, lama wool, horse hair, cattle hair, feather fiber, spider silk, and the like. Examples of the regenerated fiber include rayon, polynosic, cuprammonium, fibers containing chitin and/or chitosan, and the like. Examples of the semi-synthetic fiber include acetate, triacetate, promix, and the like. Examples of the synthetic fiber include nylon, polyester, acrylic, polyvinyl chloride, vinylon, polypropylene, polyurethane, vinylidene, polyethylene, polychlal, and the like.

In order to allow the proteoglycan to be readily covalently bonded to the fiber, the fiber is preferably a fiber having a functional group which can react with an amino or carboxyl group to bond directly to the proteoglycan. The fiber which is preferable in terms of such a point is a cellulose-based fiber derived from cellulose, specifically including: plant fibers; regenerated fibers, such as rayon, polynosic, cuprammonium, fibers blended with chitin and/or chitosan, and the like; and blended yarn of a synthetic fiber (e.g., nylon, polyester, acrylic, polyvinyl chloride, vinylon, polypropylene, polyurethane, vinylidene, polyethylene, polychlal, etc.) and a plant fiber (e.g., cotton etc.). Fibers blended with chitin and/or chitosan and fibers containing cotton are more preferable.

The form of the fiber used in the present invention is not particularly limited, as long as the proteoglycan can be covalently bond thereto. Examples of the fiber form include primary processed forms, such as original yarn, yarn, strings and ropes, woven fabric, knitted fabric, lace, felt, nonwoven fabric, pile fabric, leather and fur, and the like, and secondary processed forms obtained by further processing primary processed forms. Examples of the secondary processed product include handkerchiefs, towels, dish towels, gauze, masks, gloves, oven gloves, scarfs, shawls, mufflers, coats, kimono suits, uniforms, sweaters, skirts, slacks, cardigans, sportswear, dress shirts, pajamas, shorts, lingerie, underpants, brassieres, stockings, socks, slippers, futon's shell fabrics, sheets, futon covers, pillowcases, blankets, gloves, neckties, and the like. In the present invention, the fiber product includes the above primary and secondary processed products.

The proportion of the bonding proteoglycan in the proteoglycan-bonded fiber is not particularly limited, as long as smooth and soft texture and excellent durability can be conferred to the proteoglycan-bonded fiber product including the proteoglycan-bonded fiber. For example, 0.1 to 15 µg of the proteoglycan can be bonded to the fiber per square centimeter of gauze. In order to confer sufficient smoothness and softness to the fiber, the proteoglycan content is preferably 1 to 10 µg, and more preferably 5 to 8 µg.

The covalent bond between the fiber and the proteoglycan can be achieved by using a known technique which is suitably selected, depending on the types of the fiber and proteoglycan used. The form of the covalent bond is not particularly limited, as long as the bond of the proteoglycan can confer excellent texture and durability to the fiber product. Preferably, the covalent bond is one through which the proteoglycan and the fiber are directly bonded together without mediation of a cross-linking agent or a linker. Because of the absence of a cross-linking agent or a linker, smoother and softer texture can be conferred to the fiber product, and the fiber product additionally having excellent durability can be obtained.

Proteoglycans have an amino or carboxyl group at a terminal of the core protein, and also have a carboxyl group on a sugar chain. Therefore, by providing on the fiber a functional group which can react with any of these groups to form a bond with that group, the proteoglycan and the fiber can be bonded together. If the fiber inherently has a functional group which can react with an amino or carboxyl group, that functional group can be used to bond the proteoglycan and the fiber together. A functional group can be formed on the fiber by using a suitably selected technique known in the art.

For example, if the fiber is a cellulose-based fiber, the direct covalent bond between the cellulose-based fiber and the proteoglycan can be preferably formed as follows: an aldehyde group is formed by opening the ring of any glucose molecule included in the cellulose fiber, and the aldehyde group and an amino group of the proteoglycan are subjected to a condensation reaction. When the covalent bond is formed in such a scheme, any amino group present on the proteoglycan may be used. In order to facilitate the formation of the bond, an amino group located at the N-terminal of the core protein of the proteoglycan is preferably used.

Thus, the covalent bond between the cellulose-based fiber and the proteoglycan is preferably one which is formed by a reaction between (a) an amino group of the core protein of the proteoglycan and (b) an aldehyde group of an acyclic glucose included in the cellulose-based fiber, at least one glucose molecule included therein being an acyclic glucose molecule. Note that the acyclic glucose is a glucose molecule whose pyranose ring is opened.

The formation of an aldehyde group by opening a glucose molecule included in the cellulose-based fiber can be achieved by a suitably selected technique known in the art, preferably using an oxidant. The oxidant used is not particularly limited, as long as an aldehyde group can be formed by opening the pyranose ring. A known oxidant can be suitably selected and used. Examples of the oxidant include perchloric acid and metal salts thereof, periodic acid and metal salts thereof, potassium permanganate, manganese dioxide, chromic acid and chromates (PCC, PCD, etc.), osmium tetroxide, cerium nitrate, and the like. Metal salts are, for example, salts with an alkali metal or an alkaline-earth metal. The oxidant is preferably periodic acid or periodates (e.g., sodium periodate, potassium periodate, etc.).

The treatment of opening the pyranose ring of a glucose molecule included in the cellulose-based fiber is preferably performed under reaction conditions which do not cause damage to the fiber. For example, the cellulose fiber or the cellulose fiber product is immersed in a solution containing an oxidant (e.g., periodic acid), and allowed to react for about 0.15 to 20 h. If the reaction time is excessively short, the reaction is likely to be insufficient. If the reaction time is excessively long, the fiber is likely to be damaged by the oxidant. The concentration of the oxidant in the solution as used herein is not particularly limited, and can be, for example, 0.01 to 0.2 M, preferably 0.05 to 0.1 M. The ring-opening reaction due to the oxidant is typically performed at 0 to 50°C, preferably 20 to 30°C. The solvent for dissolving the oxidant is not particularly limited, and is typically water.

The pyranose ring of a glucose molecule included in cellulose can also be opened by immersing the cellulose fiber in an aqueous solution and achieving chemical equilibrium. When chemical equilibrium is used to open the ring, the ring can be opened, for example, by immersing cellulose in an aqueous solution at about room temperature for several hours.

The proportion of the proteoglycan bonded to the cellulose-based fiber can be adjusted by changing conditions for the treatment with the oxidant. For example, by allowing the reaction to proceed for a long time or at a high temperature, the rings of a larger number of glucose residues included in the cellulose-based fiber can be opened, so that a larger number of proteoglycans can be bonded to the fiber. On the other hand, it is considered that if the rings of a large number of glucose residues are opened, the fiber becomes brittle. Therefore, with these points in mind, conditions (the reaction time, the oxidant concentration, etc.) suitable for the intended properties of the fiber are suitably selected.

The bonding reaction between the aldehyde group of a glucose molecule which is the structural unit of cellulose and an amino group of the proteoglycan can be performed by any selected technique known in the art, preferably in the presence of a reducing agent. The reducing agent used herein is not particularly limited, as long as the aldehyde group and the amino group can be bonded together. Example of the reducing agent include hydrogenation reducing agents (e.g., sodium borohydride, lithium borohydride, lithium aluminum hydride, lithium bis(2-methoxyethoxy)aluminum hydride, lithium tri-tert-butoxy aluminum hydride, sodium cyanoborohydride, sodium borohydride, sodium cyanoborohydride, sodium triacetyloxyborohydride, etc.) or mixtures of these hydrogenation reducing agents, catalytic hydrogenation reducing agents (e.g., palladium black, palladium on carbon, platinum oxide, platinum black, Raney nickel, etc.), and the like.

The bonding reaction between the proteoglycan and the cellulose fiber having an aldehyde group in the presence of a reducing agent is not particularly limited, as long as the cellulose fiber is not damaged. For example, the bonding reaction can be performed by immersing the proteoglycan and the cellulose fiber having an aldehyde group in a solution in which the reducing agent is dissolved, for about 1 to 4 h. In this case, shaking may be performed in order to accelerate the reaction. The cellulose fiber can be immersed about one night or longer as long as the cellulose fiber is not damaged. The concentration of the reducing agent in the solution as used herein is, for example, 0.01 to 1 M, preferably 0.1 to 0.5 M. The reduction reaction can be typically performed at 0 to 40°C. The solvent in which the reaction is performed is not particularly limited, and is typically water. The concentration of the proteoglycan used in the bonding reaction is not particularly limited, as long as smooth texture and softness can be conferred to the resulting product, and can be, for example, 0.1 to 1.5 wt%.

The bonding reaction can be performed in two steps, i.e., immersion in a proteoglycan solution followed by immersion in a reducing agent solution. In this case, the concentration of the proteoglycan in the proteoglycan solution can be, for example, 0.1 to 1.5 wt%, the immersion time can be 30 min to 4 h, and the reaction temperature can be 0 to 40°C. As in the foregoing, the concentration of the reducing agent in the reducing agent solution can be 0.01 to 1 M, preferably 0.1 to 0.5 M, the immersion time can be 1 h to 4 h, and the temperature can be 0 to 40°C. The immersion times in the proteoglycan solution and the reducing agent solution for the two-step reaction are not limited, as long as the fiber is not damaged, and can also be, for example, about one night.

The proteoglycan-bonded fiber product of the present invention may have a structure in which two or more layers of proteoglycans are provided on the fiber product. The fiber product obtained by the above-described technique, to which the proteoglycan is bonded, has a large number of carboxyl groups or hydroxyl groups possessed by the core protein of the proteoglycan. Therefore, by bonding additional proteoglycans using these carboxyl groups or hydroxyl groups, a fiber product to which two or more layers of proteoglycans are bonded can be manufactured. For example, a fiber product to which two or more proteoglycan layers are bonded can be obtained by linking a carboxyl group (a carboxyl group of the core protein or a carboxyl group of a sugar chain) of the proteoglycan already present on the fiber product and an amino group of an additional proteoglycan together. This linkage can be achieved by a commonly used technique known as an amino acid condensation reaction (e.g., use of a condensation agent), and is not particularly limited.

For example, the fiber product to which a proteoglycan is bonded using the above-described technique (the fiber product to which one proteoglycan layer is bonded) can be treated with a carbodiimide compound to activate a carboxyl or alcohol group derived from the proteoglycan, and the activated carboxyl or alcohol group can then be caused to react with a proteoglycan, resulting in the fiber product to which two or more proteoglycan layers are bonded.

The treatment of bonding an additional proteoglycan to the proteoglycan layer can be repeatedly performed until the desired quality is obtained. Thus, if the treatment of bonding a proteoglycan (PG) to the fiber is repeated one or more times (preferably, a plurality of times), a fiber product (cotton etc.) to which multiple PG layers are bonded can be obtained, so that the texture is further improved than when a single proteoglycan layer is bonded, and the function of giving moisture to the skin can be effectively exhibited.

The carbodiimide compound which can be used to form two or more proteoglycan layers is not particularly limited, and can be any compound that has a functional group -N=C=N-. Examples of the carbodiimide compound include dicyclocarbodiimide, diisopropylcarbodiimide, 1-ethyl-3-(3-dimethylaminopropyl)carbodiimide hydrochloride, diisopropylcarbodiimide, and the like. A carbodiimide compound which is water-soluble and is easy to handle is preferable.

Moreover, when the reaction is performed using the carbodiimide compound, HOBt (1-hydroxybenzotriazole) as a catalyst can be used. The conditions for the reaction activated by the carbodiimide compound are not particularly limited, as long as the proteoglycan bonded to the fiber is activated by the carbodiimide compound. For example, as the reaction conditions, the concentration of the carbodiimide compound can be 0.01 to 0.2 M, the reaction temperature can be 0 to 40°C, and the reaction time can be 30 min to 60 min.

The thus-manufactured cellulose-based fiber to which the proteoglycan is directly covalently bond can be recovered from the solution, followed by thorough washing and drying, to obtain a proteoglycan-bonded fiber product. An optional additional treatment(s) can be performed on that proteoglycan-bonded fiber product to obtain a proteoglycan-bonded fiber product.

The proteoglycan can be similarly bonded to a fiber other than the cellulose-based fiber using a suitably selected known technique to produce a proteoglycan-bonded fiber product according to the present invention.

The bond of the proteoglycan to the fiber can be measured, for example, as follows. The fiber to which the proteoglycan is bonded using the above-described technique is treated using chondroitinase in an aqueous solution. After the fiber is recovered, the amount of uronic acid in the aqueous solution is measured. The free uronic acid in the aqueous solution is derived from the proteoglycan bonded to the fiber. Therefore, by detecting the uronic acid, the bond of the proteoglycan to the fiber can be confirmed.

In the present invention, other components may be bonded to the fiber as long as the function of the proteoglycan which improves the texture of the fiber product is not interfered with. Examples of such other components include hyaluronic acid, chondroitin sulfate, glycogen, dextrin, dextran, dextran sulfate, alginate, chitin, chitosan, and the like.

The proteoglycan-bonded fiber product of the present invention includes various fiber products including a fiber to which a proteoglycan is bonded. Specific examples of the proteoglycan-bonded fiber product of the present invention include yarn, strings and ropes, woven fabric, knitted fabric, nonwoven fabric, pile fabric, leather, fur, and the like, and in addition, handkerchiefs, towels, dish towels, gauze, masks, gloves, oven gloves, scarfs, shawls, mufflers, lap robes, coats, kimono suits, uniforms, shirts, blouses, aprons, kappogis (Japanese aprons), sweaters, skirts, slacks, cardigans, sportswear, dress shirts, pajamas, shorts, lingerie, underpants, brassieres, stockings, socks, tabis (Japanese socks), slippers, futon's shell fabrics, sheets, futon covers, pillowcases, tablecloths, place mats, swimsuits, furosikis (Japanese wrapping clothes), obis (Japanese sashes), curtains, blankets, gloves, neckties, and the like which are obtained by further processing the foregoing materials.

The present invention will be described below with reference to examples.

### Example 1: Manufacture of Proteoglycan-Bonded Fiber

### Product

Cotton gauze which is a cellulose-based fiber was cut into a size of 5 cm x 5 cm having a weight of about 2 g, followed by washing with water. This was immersed in 5 ml of an aqueous solution containing 0.05, 0.1 or 0.2 M NaIO₄ for 0.3 h, 8 h or 20 h at 37°C, followed by washing with water. This gauze was immersed for 1 h in an aqueous solution (5 ml) in which 0.5 wt% of a salmon cartilage-derived proteoglycan (aggrecan) had been dissolved, followed by addition of 200 mg of 0.1 M NaBH₄ to the aqueous solution. This was allowed to stand overnight, and then the gauze was recovered, followed by washing with water five to seven times and drying in the shade, to obtain gauze as a proteoglycan-bonded fiber product.

### Test Example 1: Texture Test

The feel in use of the proteoglycan-bonded gauze produced in Example 1 was tested with 21 adult participants. The participants used the gauze and expressed their opinions or feelings which are summarized in Table 1. Untreated gauze as control was similarly evaluated. Note that, in the evaluation, each participant was allowed to evaluate the gauze on multiple subjects.

**Table 1**

| Evaluation content | Number of respondents |
|---|---|
| Smooth and comfortable texture | 20 |
| Soft feel remains on skin after use | 16 |
| Smooth feel remains on skin after use | 16 |
| Moist feel is present on skin after use | 3 |
| Texture similar to when fabric softener is used, but no smell typical of fabric softener | 3 |
| Not very different from control | 1 |

It can be seen from the results of Table 1 that, by bonding a proteoglycan directly to the fiber, the fiber product has considerably excellent texture (smoothness and softness), and in addition, a groundbreaking function of conferring moist feel to the skin.

### Example 2: Manufacture of Proteoglycan-Bonded Hand

### Towel

A hand towel to which a proteoglycan is bonded was produced by a manufacturing method similar to that of Example 1, except that the hand towel was produced instead of gauze.

### Test Example 2: Measurement of Durability

The proteoglycan-bonded hand towel manufactured in Example 2 was repeatedly washed and dried using a household washing machine and a household detergent. Each time washing and drying were performed, the resulting texture was evaluated by comparing it with that before washing. The results are shown in Table 2.

**Table 2**

| Number of times of washing and drying | Evaluation in terms of texture |
|---|---|
| 0 | Soft and very good texture |
| 1 to 3 | Not different from before washing |
| 4 to 8 | Slightly lesser texture than before washing, but significantly better texture than towel to which proteoglycan is not bonded |
| 9 to 15 | Softness gradually decreases, but sufficient softness is maintained. Significantly softer than towel to which proteoglycan is not bonded. Damage to towel fiber is not observed |

The results of Table 2 show that the fiber product in which a proteoglycan is bonded directly to the fiber has texture which is not significantly degraded even after repeated washing and drying, and therefore, has excellent durability.

### Example 3: Manufacture of Proteoglycan-Bonded Towel and

### T-Shirt

A cotton towel of 33 cm (length) x 33 cm (width) and a cotton T-shirt were used to manufacture a towel and a T-shirt to which a proteoglycan was bonded, using a procedure described below.

Ten cotton towels and ten cotton T-shirts were treated by immersion in 3 liters of a 0.1 M NaIO₄ solution (sodium periodate solution) at room temperature for 10 min. The treated cotton towels and cotton T-shirts were well squeezed to remove the sodium periodate solution, hand-washed with tap water, and well squeezed. Next, the cotton towels and the cotton T-shirts were immersed in 3 liters of a 1 wt% proteoglycan solution, and allowed to stand at room temperature overnight. Thereafter, the cotton towels and the cotton T-shirts were well squeezed to remove the proteoglycan solution, and immersed in a 0.1 M NaBH₄ solution at room temperature overnight. The cotton towels and the cotton T-shirts were sometimes stirred during the immersion in the NaBH₄ solution. After the immersion overnight, the cotton towels and the cotton T-shirts were removed, and hand-washed with water. Thereafter, the cotton towels and the cotton T-shirts were washed with water twice using a washing machine. The reason why a washing machine is used after the hand-washing is that the cotton towels and the cotton T-shirts are thoroughly washed with water. Finally, water was removed from the cotton towels and the cotton T-shirts using a washing machine, followed by air drying in the room.

### Example 4

A procedure similar to that of Example 3 was performed, where the treatment with 0.1 M NaIO₄ (sodium periodate solution) was performed for different times 20 min, 30 min, and 50 min. Fiber product samples used were 33 x 33 cm cotton towels (total number: 30), 23 x 23 cm cotton towels (total number: 30), cotton gauze towels (total number: 30), cotton cloth, and cotton. The immersion in the 0.1 M sodium periodate solution was performed at room temperature for 50 min. The treatment with the 1 wt% proteoglycan solution was performed for 4 h. The reduction with 0.1 M NaBH₄ was performed at room temperature for 4 h. Damage was not observed for any of the fiber product samples.

### Example 5

A proteoglycan-bonded cotton towel was produced by a procedure similar to that of Example 3, except that the treatment with the 0.1 M sodium periodate solution was performed for 30 min, the treatment with 1 wt% proteoglycan was performed for 4 h, and the treatment with 0.1 M NaBH₄ was performed for 4 h. A proteoglycan was additionally bonded to the cotton towel by a procedure described below.

The proteoglycan-bonded cotton towel was immersed and activated in a 0.02 M 1-ethyl-3-(3-dimethyl aminopropyl)carbodiimide hydrochloride solution at room temperature for about 30 min, and then removed and well squeezed. The cotton towel was immersed in a 1 wt% proteoglycan solution at room temperature for about 60 min. Thereafter, the cotton towel was removed from the proteoglycan solution, hand-washed with water, and squeezed to remove water, followed by air drying in the room.

A texture test was performed on the resulting cotton towel. As a result, an improvement in smooth feel was observed for the cotton towel (PG-PG fiber) which was subjected to the proteoglycan bonding treatment twice.

### Test Example 3: Texture Test

A texture test was performed on each of the proteoglycan-bonded fiber products obtained in Examples 3 and 4. Fifteen adult participants were randomly selected, and asked to dry their wet hands on the proteoglycan-processed cotton cloth. As a result, 14 of the participants expressed their opinions or feelings that there are good smooth feel and gentle feel to the skin, such as "after wet hands are dried on this PG-bonded fiber, smooth feel remains on the skin," and the like. The remaining one participant expressed his or her opinion or feeling that "I do not get those impressions." This result shows that the proteoglycan-bonded cotton cloth has excellent texture, such as gentle texture to the skin and the like.

Also, when the proteoglycan-bonded cotton cloth (cotton towel) was wet and used as a wet towel, an opinion or feeling that smooth feel remains on the hand was obtained. This result shows that even when the cotton towel is wet, the cotton towel has good texture.

Moreover, the participants were asked to wear the proteoglycan-bonded T-shirt during sleep, and on the next morning, the state of the skin was evaluated. As a result, an opining or feeling was expressed that the state of the skin which had been in contact with the T-shirt provided smoother and more moist feel than that of the state of the skin which had not been in contact with the T-shirt.

### Test Example 4: Durability Test

A proteoglycan-bonded fiber product was repeatedly washed to investigate the durability of the bonding proteoglycan.

A cotton towel and a cotton gauze towel to which a proteoglycan was bonded were produced by a procedure similar to that of Example 3, except that the treatment with 0.1 M sodium periodate was performed for 30 min, the treatment with 1 wt% proteoglycan was performed for 4 h, and the treatment with 0.1 M NaBH₄ was performed for 4 h, and were washed three times. The resulting cotton towel and cotton gauze towel were subjected to a test described below.

Ten cotton towels and ten cotton gauze towels were washed with a detergent and rinsed using a washing machine. After the washing and rinsing five times, water was removed from six of each ten, which were then hung and dried in the room. The rest of the samples (four of each ten) were washed with a detergent and rinsed additional five times using the washing machine. After the washing and rinsing a total of 10 times using the washing machine, water was removed from the cotton towels and the cotton gauze towels, which were then dried in the room.

A texture test was performed on the thus-treated cotton towels and cotton gauze towels. As a result, smooth feel of the cotton towels and the cotton gauze towels was well maintained even after the washing treatment was performed five and ten times. This suggests that the proteoglycan-bonded fiber product of the present invention has excellent durability even against washing using a washing machine because a proteoglycan is stably bonded to the fiber.

## Claims

1. A proteoglycan-bonded fiber product comprising a fiber to which a proteoglycan is covalently bonded.

2. The proteoglycan-bonded fiber product of claim 1,
wherein the fiber is a cellulose-based fiber.

3. The proteoglycan-bonded fiber product of claim 2,
wherein the covalent bond is formed by a reaction of:
(a) an amino group of a core protein of the proteoglycan; and
(b) an aldehyde group of at least one acyclic glucose constituting the cellulose-based fiber.

4. The proteoglycan-bonded fiber product of any of claims 1 to 3, wherein the proteoglycan is aggrecan.

5. A method for manufacturing a proteoglycan-bonded fiber product, comprising the step of covalently bonding a proteoglycan to a fiber.

6. The method of claim 5, wherein the step of covalently bonding a proteoglycan to a fiber includes the sub-steps of:
(1) treating a cellulose-based fiber with an oxidant to form an aldehyde group on a glucose molecule constituting the cellulose fiber; and
(2) reacting the cellulose-based fiber having the aldehyde group obtained by step (1) with the proteoglycan in the presence of a reducing agent.

7. The method of claim 6, further comprising the step of bonding, one or more times, an additional proteoglycan to the proteoglycan of the proteoglycan-bonded fiber product obtained by sub-step (2).
